# EUROPEAN PATENT SPECIFICATION

(11) **EP 1 229 909 B1**
(45) Date of publication and mention of the grant of the patent: **12.11.2003**
(21) Application number: 01939751.2
(22) Date of filing: 31.05.2001
(51) Int. Cl.: A61K 31/40, A61K 9/70, A61P 5/30

(54) **TRANSDERMAL DELIVERY OF LASOFOXIFENE**
TRANSDERMALE VERABREICHUNG VON LASOFOXIFEN
ADMINISTRATION TRANSCUTANEE DE LASOFOXIFENE

(30) Priority: 01.06.2000 US 208789 P
(43) Date of publication of application: 14.08.2002
(73) Proprietor: Watson Pharmaceuticals, Inc., Corona, CA 92880 (US)
(72) Inventor: FIKSTAD, David, Salt Lake City, UT 84102 (US); QUAN, Danyi, Salt Lake City, UT 84107 (US)
(74) Representative: Brasnett, Adrian Hugh
(86) International application number: US0117567
(87) International publication number: WO01091724

(56) References cited:
- EP-A- 1 057 480
- WO-A-96/21656

## Description

### TECHNICAL FIELD

This invention relates to the transdermal delivery of lasofoxifene (5-substituted-6-cyclic-5,6,7,8,-tetrahydronaphthalene2-ol) compounds.

### BACKGROUND OF THE INVENTION

Naturally occurring estrogens and synthetic compositions demonstrating "estrogenic" activity are useful for various therapeutic applications for example, oral contraception; relief for the symptoms of menopause; prevention of threatened or habitual abortion; relief of dysmenorrhea; relief of dysfunctional uterine bleeding; aiding in ovarian development; treating acne; diminution of excessive growth of body hair in women (hirsutism); the preventing cardiovascular disease; treating osteoporosis; treating prostatic carcinoma; and suppressing post-partum lactation [Goodman and Gilman, The Pharmacological Basis Of Therapeutics (Seventh Edition) Macmillan Publishing Company, 1985, pages 1421-1423]. Accordingly, there has been increasing interest in finding newly synthesized compositions and new uses for previously known compounds that are demonstrably estrogenic, this is, able to mimic the action of estrogen in estrogen responsive tissue. From the viewpoint of pharmacologists interested in developing new drugs useful for the treatment of human diseases and specific pathological conditions, it is most important to procure compounds with some demonstrable estrogen-like function but which are devoid of proliferative side-effects. For example, osteoporosis, a disease in which bone becomes increasingly, more fragile, is greatly ameliorated by the use of fully active estrogens; however, due to the recognized increased risk of uterine cancer in patients chronically treated with active estrogens, it is not clinically advisable to treat osteoporosis in intact women with fully active estrogens for prolonged periods. Estrogen is the agent of choice in preventing osteoporosis or post menopausal bone loss in women; it is the only treatment which unequivocally reduces fractures. However, estrogen stimulates the uterus and is associated with an increased risk of endometrial cancer. Although the risk of endometrial cancer is thought to be reduced by a concurrent use of a progestogen, there is still concern about possible increased risk of breast cancer with the use of estrogen.

Estrogen and estrogen-like compounds have also been shown to lower plasma levels of LDL and raise those of the beneficial high density lipoproteins (HDL's). Black, et al. in EP 0605193A1. Long-term estrogen therapy, however, has been implicated in a variety of disorders, including an increase in the risk of uterine cancer and possibly breast cancer, causing many women to avoid this treatment. Recently suggested therapeutic regimens, which seek to lessen the cancer risk, such as administering combinations of progestogen and estrogen, cause the patient to experience unacceptable bleeding. Furthermore, combining progesterone with estrogen seems to blunt the serum cholesterol lowering effects of estrogen. The significant undesirable effects associated with estrogen therapy support the need to develop alternative therapies for hypercholesterolemia that have the desirable effect on serum LDL but do not cause undesirable effects.

Lasofoxifene (CP-336,156) is a selective estrogen receptor modulator (agonist/antagonist). It has been shown to have similar therapeutic effects in bone and LDL levels to estradiol but without the uterine-stimulating effects associated with estradiol therapy. Ke H.Z. (1998) Endocrinology 139(4):2068-2076 and Roasti, R.L. (1998) J. Med. Chem. 41(16):2928-2931. It also has been shown to prevent bone loss in ovariectomized rats and postmenopausal women. Zhu Ke, H. (2000) Endocrinology 141(4):1338-1344. The latter study also reports that lasofoxifene decreased total serum cholesterol in female and male rats and did not affect prostate in the male rats. Thus, there is an established therapeutic benefit for the oral administration of lasofoxifene.

In certain situations, however, oral administration of drugs is unsatisfactory. For drugs with short half lives require frequent dosing (2 to 4 times daily), may lead to inadequate compliance by the patient. Second, the short plasma half life of the drug and frequent dosing regimen result in "peaks" and "valleys" in the plasma concentration profile, which increases the likelihood of adverse side effects associated with the peak concentration as well as lapse of therapeutic effectiveness toward the end of the dosing interval. Third, the potential effect of hepatic first pass metabolism associated with oral administration could lead to poor bioavailibility of the drug. Thus, an effective and consistent drug delivery system that overcomes these disadvantages would be far advantageous.

Transdermal delivery of drugs provides many advantages over conventional oral administration. Advantages of transdermal systems include convenience, uninterrupted therapy, improved patient compliance, reversibility of treatment (by removal of the system from the skin), elimination of "hepatic first pass" effect, a high degree of control over blood concentration of the drug, and improved overall therapy.

### DISCLOSURE OF THE INVENTION

The present invention provides pharmaceutical formulations, and devices for the transdermal delivery of 5-substituted-6-cyclic-5,6,7,8,-tetrahydronaphthalene2-ol compounds ("lasofoxifene" or "CP-336,156") and pharmaceutically acceptable salts thereof as defined in the appended claims. Also described herein are transdermal compositions of CP-336,156 or its salts dissolved or dispersed in a suitable carrier vehicle, optionally containing permeation enhancers and other excipients. The carrier vehicle may be a pressure sensitive adhesive, polymeric reservoir, or a fluid of controlled viscosity. The carrier vehicle may be contained in a device for purposes of holding the composition against the skin surface. Such devices may be in the form of matrix patches (drug in adhesive) or reservoir patches (drug in a liquid or polymeric reservoir with peripheral, in-line, or over-layed pressure sensitive adhesive). Also described herein is the use of the pharmaceutical formulations of the present invention for the preparation of a medicament for treating pathologies associated with the binding of lasofoxifene with the human estrogen receptor-alpha. For example, the invention formulations and devices are useful to treat or prevent bone loss, obesity, breast cancer, endometriosis, cardiovascular disease and prostatic disease.

### MODES FOR CARRYING OUT THE INVENTION

In describing and claiming the present invention, the following terminology will be used in accordance with the definitions set out below.

As used in the specification and claims, the singular form "a," "an" and "the" include plural references unless the context clearly dictates otherwise. For example, the term "a cell" includes a plurality of cells, including mixtures thereof.

As used herein, the term "comprising" is intended to mean that the compositions and methods include the recited elements, but not excluding others. "Consisting essentially of" when used to define compositions and methods, shall mean excluding other elements of any essential significance to the combination. Thus, a composition consisting essentially of the elements as defined herein would not exclude trace contaminants from the isolation and purification method and pharmaceutically acceptable carriers, such as phosphate buffered saline, preservatives, and the like. "Consisting of" shall mean excluding more than trace elements of other ingredients and substantial method steps for administering the compositions of this invention. Embodiments defined by each of these transition terms are within the scope of this invention.

As used herein, the term "lasofoxifene" is synonymous with "CP-336,156" and "5-substituied-6-cyclic-5,6,7,8,-tetrahydronaphthalene2-ol" and pharmaceutical acceptable salts thereof. The preparation of lasofoxifene and its pharmaceutical acceptable salts is disclosed in U.S. Patent No. 5,552,412. The term "lasofoxifene" intends the compounds and formulations disclosed in U.S. Patent No. 5.552.412.

As used herein, the terms "enhancement", "penetration enhancement" or "permeation enhancement" mean an increase in the permeability of a biological membrane (i.e. skin or mucosa) to a drug, so as to increase the rate at which the drug permeates through the membrane. "Permeation enhancer," "enhancer," "penetration enhancer," or similar term means a material that achieves such permeation enhancement, and an "effective amount" of an enhancer means an amount effective to enhance penetration through the skin or mucosa of a selected agent to a selected degree. The enhanced permeation as effected though the use of such enhancers can be observed, for example, by measuring the rate of diffusion of the drug through animal or human skin using a diffusion cell apparatus. Such a diffusion cell is described by Merritt et al., Diffusion Apparatus for Skin Penetration, 1 J. of Controlled Release 61 (1984).

As used herein, "transdermal" or "percutaneous" delivery means delivery of a drug by passage into and through the skin or mucosal tissue. Hence the terms "transdermal" and "transmucosal" are used interchangeably unless specifically stated otherwise. Likewise the terms "skin," "derma," "epidermis," "mucosa," and the like shall also be used interchangeably unless specifically stated otherwise.

By "effective amount" of a drug or permeant is meant a nontoxic but sufficient amount of a compound to provide the desired local or systemic effect. An "effective amount" of permeation enhancer as used herein means an amount selected so as to provide the desired increase in membrane permeability and, correspondingly, the desired depth of penetration, rate of administration, and amount of drug.

By "drug delivery system," "drug/enhancer composition," or any similar terminology is meant a formulated composition containing the drug to be transdermally delivered in combination with a penetration enhancer. Other pharmaceutically acceptable materials or additives can also be contained in the drug/enhancer composition, such as a diluent, skin-irritation reducing agent, carrier or vehicle, excipient, plasticizer, emollient, or other additive and mixtures thereof provided that such additives do not materially affect the basic and novel characteristics of the matrix patch.

By the term "matrix," "matrix system," or "matrix patch" is meant an active permeant or drug dissolved or suspended in a biocompatible polymeric phase, preferably a pressure sensitive adhesive, that can also contain other ingredients or in which the enhancer is also dissolved or suspended. This definition is meant to include embodiments wherein such polymeric phase is laminated to a pressure sensitive adhesive or used with an overlay adhesive. A matrix system usually and preferably comprises an adhesive layer having an impermeable film backing laminated onto the distal surface thereof and, before transdermal application, a release liner on the proximal surface of the adhesive. The film backing protects the polymeric phase of the matrix patch and prevents release of the drug and/or enhancer to the environment. The release liner functions similarly to the impermeable backing, but is removed from the matrix patch prior to application of the patch to an application situs. Matrix patches are known in the art of transdermal drug delivery to routinely contain such backing and release liner components, and matrix patches according to the present invention should be considered to comprise such backing and release liner or their functional equivalents. U.S. Pat. No. 5,122,383 describes such backing and release liner. A matrix system therefore is a unit dosage form of a drug composition in a polymeric carrier, also containing the enhancer and other components which are formulated for maintaining the drug composition in the polymeric layer in a drug transferring relationship with the derma, i.e. the skin or mucosa. A matrix patch is distinguished from a "liquid reservoir patch," wherein an active permeant or drug is dissolved in a gelled liquid contained in an occlusive device having an impermeable back surface and an opposite surface configured appropriately with a permeable membrane and adhesive for transdermal application, e.g., U.S. Pat No. 4,983,395.

As used herein, "application situs" means a site suitable for topical application with or without the means of a device, patch, or dressing, e.g. behind the ear, on the arm, back, chest, abdomen, leg, top of foot, etc.

A "composition" is intended to mean a combination of active agent and another compound or composition, inert (for example, a detectable agent or label) or active, such as an adjuvant.

A "pharmaceutical composition" is intended to include the combination of an active agent with a carrier, inert or active, making the composition suitable for diagnostic or therapeutic use *in vitro, in vivo* or *ex vivo*.

As used herein, the term "pharmaceutically acceptable carrier" encompasses any of the standard pharmaceutical carriers, such as a phosphate buffered saline solution, water, and emulsions, such as an oil/water or water/oil emulsion, and various types of wetting agents. The compositions also can include stabilizers and preservatives. For examples of carriers, stabilizers and adjuvants, see Martin REMINGTON'S PHARM. SCL, 15th Ed. (Mack Publ. Co., Easton (1975)).

A "subject" is a vertebrate, preferably a mammal, more preferably a human. Mammals include, but are not limited to, marines, simians, humans, farm animals, sport animals, and pets.

To "treat" means to alleviate the symptoms or modify clinical manifestation of a disease or condition. To "prevent" means to delay or minimize the symptoms or clinical manifestations of a disease or condition. For the purpose of this invention, diseases or conditions suitably treated by this invention are those associated with the binding of the estrogen receptor by its natural ligand. Such conditions include, but are not limited to obesity, breast cancer, osteoporosis, endometriosis, cardiovascular disease, prostatic disease, ovulation, and blood cholesterol levels, especially LDL serum levels.

In its most basic form, this invention provides a transdermal formulation of a drug reservoir containing an effective amount of lasofoxifene and/or a pharmaceutically acceptable salt thereof, an effective amount of a drug permeation enhancer and a cell-envelope disordering compound as defined in the appended claims. Examples of cell-envelope disrupters include but are not limited to, isopropyl myristate, methyl laurate, oleic acid, oleyl alcohol, glycerol monooleate, glycerol dioleate, glycerol trioleate, glycerol monostearate, glycerol monolaurate, propylene glycol monolaurate or sorbitan esters. See U.S. Patent No. 5,626,866. In addition formulation may also contain one or more skin permeation enhancers such as triacetin. Examples of enhancers that may be used, without limitation, include saturated and unsaturated fatty acids and their esters, alcohols, monoglycerides, acetate, diethanolamides and N, N-dimethylamides, such as oleic acid, propyl oleate, isopropyl myristate, glycerol monooleate, glycerol monolaurate, methyl laurate, lauryl alcohol, lauramide diethanolamide and combinations thereof. Saturated and unsaturated sorbitan esters, such as sorbitan monooleate and sorbitan monolaurate may also be used.

In one aspect, the drug reservoir is an adhesive matrix which can be water based or solvent based. The adhesive matrix may have the additional characteristic of being pressure sensitive suitable for long-term contact with the skin. Such adhesives must be physically and chemically compatible with lasofoxifene and the enhancer, and with any carriers and/or vehicles or other additives incorporated into the drug/enhancer composition. Suitable adhesives for use in the matrix patches include acrylic adhesives including cross-linked and uncross-linked acrylic copolymers; vinyl acetate adhesives; natural and synthetic rubbers including polyisobutylenes, neoprenes, polybutadienes, and polyisoprenes; ethylenevinylacetate copolymers; polysiloxanes; polyacrylates; polyurethanes; plasticized weight polyether block amide copolymers, and plasticized styrene-rubber block copolymers

Suitable pressure sensitive adhesives include polysiloxanes, polyacrylates, polyisobutylene, and the like. These pressure sensitive adhesive polymers are very hydrophobic and are typically purchased as solutions of polymer dissolved in organic solvents. The drug and selected excipients, if any, are directly incorporated into the organic-solvent-based pressure sensitive adhesive solution, mixed, cast as a thin film, and dried to evaporate the solvents, leaving a dried adhesive matrix film containing the drug and excipients. It is well known in the art that the drug has to be hydrophobic to be incorporated into the organic-solvent-based, hydrophobic adhesive. Hydrophilic salt forms of a drug are generally not compatible with such organic-solvent-based pressure sensitive adhesives and have to be converted to the more hydrophobic free acid or free base form for incorporation into the organic-solvent-based, hydrophobic adhesive.

Water-based pressure sensitive adhesives are also commercially available. These water-based adhesives are formulated as emulsions wherein the hydrophobic pressure sensitive adhesive polymer is dispersed in water with the help of surfactants. Such water-based adhesives provide inherent advantages of safety and reduced environmental problems over solvent-based pressure sensitive adhesives, because the carrier is water and not an organic solvent The water-based adhesives are widely used in the manufacture of medical tapes and bandages, and provide excellent skin adhesion.

U.S. Patent Nos. 5,985,317; 5,783,208; 5,780,050; 5,626,866; 5,460,820 and 4,983,395 describe various polymeric transdermal matrix formulations.

Alternatively, the drug reservoir is a liquid reservoir as described in U.S. Patent No. 5,662,925; 4,829,224 or 4,983,395. Alternative embodiments known in the art are described in U.S. Patent No. 4, 829,224; 4,849,224 and 4,983,395.

The matrix patch can further comprise various additives in addition to the polymer layer containing lasofoxifene and enhancer, that are the fundamental components of the transdermal drug delivery system. These additives are generally those pharmaceutically acceptable ingredients that are known in the art of drug delivery and, more particularly, in the art of transdermal drug delivery provided that such additive ingredients do not materially alter the basic and novel characteristics of the matrix patch. For example, suitable diluents can include mineral oil, low molecular weight polymers, plasticizers, and the like. Many transdermal drug delivery formulations have a tendency to cause skin irritation after prolonged exposure to the skin, thus addition of a skin irritation reducing agent aids in achieving a composition that is better tolerated by the skin. A preferred skin irritation reducing agent is glycerin, U.S. Pat. No. 4,855,294. It is however notable that other so-called acceleration promoters or permeation enhancer components such as solvents are not necessary in the present invention.

The drug reservoir containing lasofoxifene may be embodied in various types of structures known in the transdermal drug delivery art. For instance, the drug reservoir, which is the most important component of the device, may comprise a simple matrix of a subsaturated solution of lasofoxifene in the carrier or be in the form of a fibrous body impregnated with the subsaturated solution of lasofoxifene in the carrier. In addition to the reservoir, the device includes means for maintaining the reservoir in drug delivery communication with the skin. Such means include a carrier which is also an adhesive, a separate basal adhesive layer underlying the reservoir, a peripheral ring of adhesive that is interconnected to the reservoir, an adhesive overlay for the reservoir, and straps. Preferably the means is either an adhesive carrier or a separate underlying adhesive layer. Preferably the device is in the form of a laminated composite.

These devices may be manufactured by conventional techniques used in the transdermal drug delivery device art. For instance the drug and carrier may be mixed in the desired proportions to form a homogeneous mix and cast or otherwise applied to a backing layer, followed by lamination to a release liner layer. If a separate basal adhesive layer is desired, it may be cast onto the release liner layer prior to such lamination.

In use, the matrix patch may contain a distal backing laminated on the polymer layer. The distal backing defines the side of the matrix patch that faces the environment, i.e., distal to the skin or mucosa. The backing layer functions to protect the matrix polymer layer and drug/enhancer composition and to provide an impenetrable layer that prevents loss of drug to the environment. Thus, the material chosen for the backing should be compatible with the polymer layer, drug, and enhancer, and should be minimally permeable to any components of the matrix patch. Advantageously, the backing can be opaque to protect components of the matrix patch from degradation from exposure to ultraviolet light. Further, the backing should be capable of binding to and supporting the polymer layer, yet should be pliable to accommodate the movements of a person using the matrix patch. Suitable materials for the backing include metal foils, metalized polyfoils, composite foils or films containing polyester such as polyester terephthalate, polyester or aluminized polyester, polytetrafluoroethylene, polyether block amide copolymers, polyethylene methyl methacrylate block copolymers, polyurethanes, polyvinylidene chloride, nylon, silicone elastomers, rubber-based polyisobutylene, styrene, styrene-butadiene and styreneisoprene copolymers, polyethylene, and polypropylene. A thickness of about 0.0005 to 0.01 inch is preferred. The release liner can be made of the same materials as the backing, or other suitable films coated with an appropriate release surface.

The drug reservoirs are applied to the application situs and the drug diffuses through the dermis. Also described herein is a drug reservoir, as described herein, and a means for adhering the reservoir to the application situs. Examples of such devices are described above and include an adhesive matrix containing the drug, a backing layer and a releasable liner. See also U.S. Patent Nos. 5,164,190 and 5,985,317.

For example, such a device includes a laminated composite of a backing layer defining an upper portion of a reservoir and extending to the periphery of a peel seal disk; an active agent-permeable membrane extending to the periphery of the peel seal disk and the backing layer, and underlying the backing layer, the backing layer and membrane defining; the reservoir therebetween that contains the formulation of this invention; the peel seal disc underlying an active agent-permeable membrane; a heat seal about the periphery of the peel seal disc, the active agent-permeable membrane and the backing layer, an adhesive overlay having a central portion overlying the backing layer and a peripheral portion that extends beyond the periphery of the peel seal disc; and a removable release liner underlying the peripheral portion of the adhesive overlay and the peel seal disc.

The above pharmaceutical formulations, drug reservoirs and devices are useful to treat or prevent a disorder associated with estrogen disregulation in a subject by contacting any of the pharmaceutical formulation, the drug reservoir or the device with the application situs of the subject.

This invention further provides use of an effective amount of lasofoxifene for the preparation of a transdermal medicament for the treatment or prevention of a disorder associated with estrogen disregulation, as defined in the appended claims.

### EXPERIMENTAL METHODOLOGY

### Adhesive Matrix Preparation

Pressure sensitive adhesive matrix systems prepared according to the teachings of U.S. Patent No. 5,952,000. First, the solids content of the adhesive solution (water or organic solvent based) was determined by placing a known weight of solution in a weighed aluminum dish and evaporating the solvents overnight in a 70. degree. C. convection oven. The solid adhesive content of the solution was calculated by dividing the adhesive solid weight after drying by the initial total solution weight. Next, a weighed quantity of adhesive solution was added to a glass bottle and the drug substance, permeation enhancer, and other excipients were weighed and added to the adhesive solution in a quantity necessary to achieve the desired dry matrix film composition. The solution containing the adhesive polymer, drug substance, and other excipients as necessary was then mixed overnight. After mixing, approximately 8 ml of the solution was dispensed on a silanized polyester release liner and film cast using a casting knife with a gap size appropriate to achieve a final dried thickness of approximately 0.05 mm. The cast film was dried in a 70.degree. C. convection oven until all solvents had evaporated to yield a dried matrix (15 minutes for organic solvent based adhesives, 30 minutes for water emulsion based adhesives). Finally, an 0.08 mm thick occlusive polyethylene backing film was laminated onto the dried adhesive matrix, and these systems were then used to conduct in vitro skin flux experiments as described below.

### Reservoir or Free Form Hydroalcoholic Gel Preparation

Hydroalcoholic gels were prepared on a 10 ml scale as follows. Ethyl alcohol (190 proof ethanol), water, glycerin, enhancer and drug were combined in the appropriate proportions and mixed for several hours. The gelling agent (hydroxypropylcellulose) was added and the solution was mixed briefly at high shear, then mixed at low shear until a gel was formed.

### Skin Flux Studies

In vitro skin flux studies were conducted using human cadaver epidermal membrane in modified Franz non-jacketed diffusion cells. The epidermal membrane (stratum corneum and epidermis) was separated from whole skin (epidermal membrane and dermis) by the method of Kligman and Christopher (Arch. Dermatol. 88:702 (1963)). This method involves the exposure of the full-thickness skin to water at 60.degree C. for a time period of 60 seconds. After this period, the epidermal membrane was gently peeled off the dermis and stored for later use in aluminum foil at -5.degree. C.

Prior to each permeation experiment with a matrix system, the matrix system was cut into a circular sample of 0.7 cm.sup.2 area and the silanized release liner was removed. The adhesive was affixed to the stratum corneum side of the thawed epidermal membrane which was then cut to an appropriate size and clamped in place between the two halves of the diffusion cell with the stratum corneum facing the donor compartment. The receiver compartment was filled with water or an aqueous solution appropriate to maintain sink conditions for the drug. All receiver solutions included 0.02% (w/w) sodium azide (NaN₃) to inhibit bacterial growth. The diffusion cell was placed in a temperature controlled circulating water bath calibrated to maintain the surface temperature of the skin at 32.degree. C. The receiver compartment was constantly stirred by a magnetic stir-bar in the receiver compartment agitated by a magnetic stirring module placed under the water bath.

Permeation experiments with hydroalcoholic gels were performed using finite occluded doses. The occluded dose is an appropriate in vitro model for the application of a transdermal patch drug delivery system containing a liquid or gel reservoir.

Occluded dosing experiments were set-up according to the following procedure. Prior to skin permeation experiments, the epidermal membrane was cut to an appropriate size and placed between the two halves of the diffusion cell with the epidermal side facing the receiver compartment. The receiver compartment was filled with an appropriate solution then the diffusion cell was placed in a circulating water bath calibrated to maintain the temperature ofthe skin surface at 32.degree. C. and allowed to hydrate overnight. After hydration, a sample of the gel (75 .mu.l) was pipetted into a cavity created by placing a polyethylene washer over the stratum corneum surface. This cavity was covered with an occlusive backing film which was clamped in place.

Permeation experiments with aqueous solutions were performed using presaturated drug solutions containing excess drug solid (infinite dose). Prior to skin permeation experiments, the epidermal membrane was allowed to hydrate over night as described above. After hydration a well mixed sample of the aqueous solution (1 ml) was pipetted into the donor compartment formed by clamping a glass lid above the stratum corneum surface. The glass lid was then sealed with a Teflon.RTM. lined polypropylene cap.

The following sampling procedure was used for all dosage forms. At predetermined sampling time points, the entire contents of the receiver compartment were collected for drug quantitation and the receiver compartment was filled with fresh solution, taking care to eliminate any air bubbles at the skin/solution interface. The cumulative amount of drug permeated per unit area at any time.

The following examples are intended to illustrate, not limit the invention.

### Example 1

A transdermal matrix formulation was prepared with a solvent-based acrylic pressure sensitive adhesive (TSR 58; Sekisui Chemical Co., Osaka, Japan), triacetin (Eastman), and CP-336,156 in the proportions 84/10/6% w/w. Results of *in vitro* skin flux experiments using this matrix formulation are summarized in Table 1.

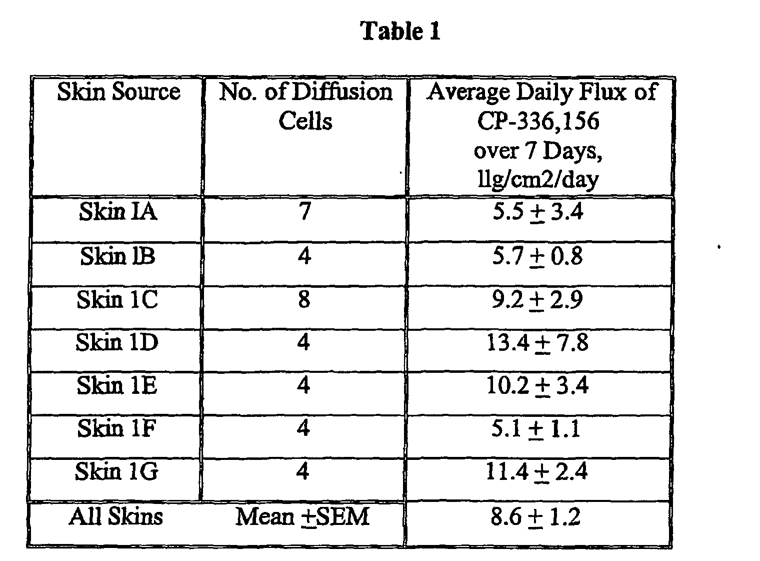

The results in Table 1 illustrate that CP-336,156 may be incorporated into a matrix patch containing triacetin as a skin permeation enhancer. Transdermal delivery of CP-336,156 from this formulation can be maintained for at least 7 days.

### Example 2

A transdermal matrix formulation was prepared in a water-based acrylic pressure sensitive adhesive (Morstik 214, Morton, Greenville, SC) with CP-336,156 tartrate salt at a concentration of 3% w/w. A permeation enhanced formulation was prepared with 3%w/w CP-336,156 tartrate salt and 1.5%w/w sodium lauroyl glycolate (R.I.T.A. Corporation, Woodstock, IL) in the same adhesive. Results of *in vitro* skin flux experiments using these datrix formulations are summarized in Table 2.

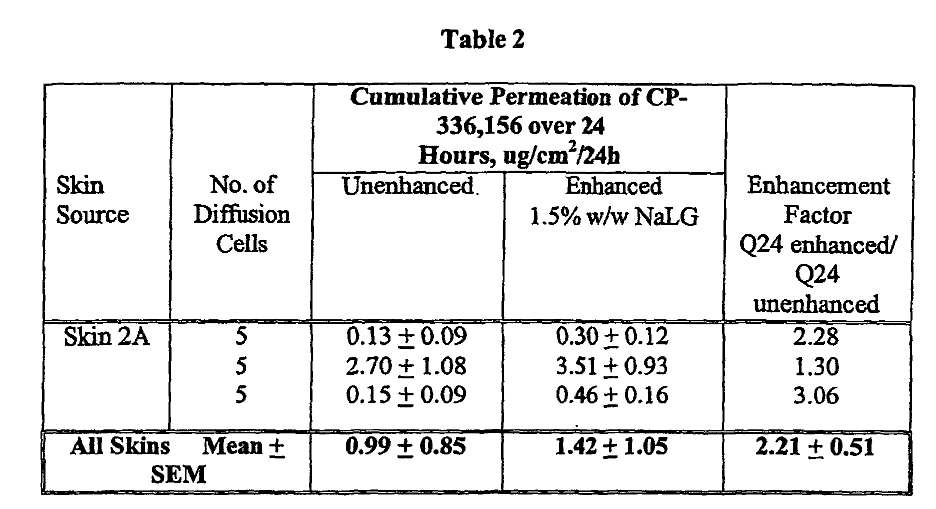

The results in Table 2 illustrate that salts of CP-336,156 may be incorporated into an adhesive matrix patch. The mean enhancement factor was 2.2 illustrating that effective amounts of a permeation enhancer may also be incorporated in these matrix systems.

### Example 3

A transdermal liquid reservoir formulation was prepared with a solvent composition of USP alcohol (EtOH), water (H20), glycerin (Gly), glycerol monooleate (GMO), and methyl laurate (ML) in the proportions 50/15/30/2.5/2.5% v/v. This mixture was a clear solution. CP-336,156 tartrate salt was added at a concentration of 2 mg/ml and the formulation was gelled with 30 mg/g hydroxypropylmethylcellulose (Methocel® E10M, Dow Chemical). Results of *in vitro* skin flux experiments on this formulation are summarized in Table 3.

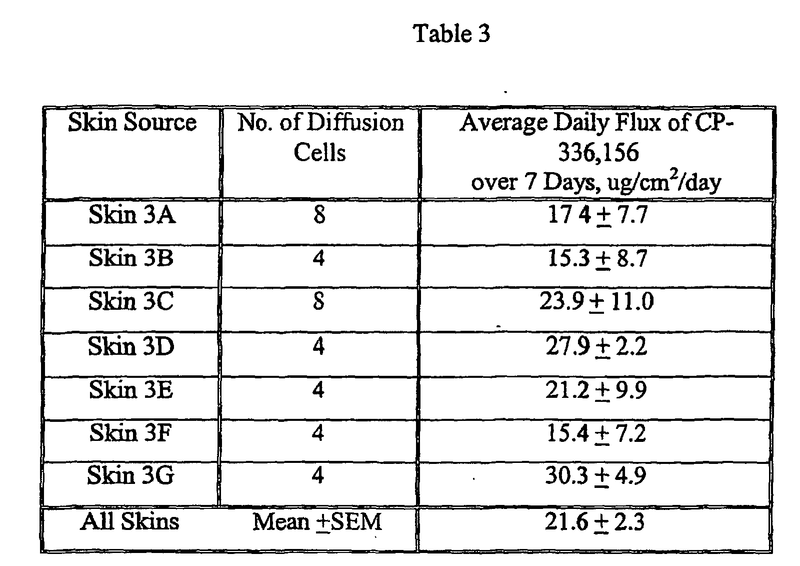

The results in Table 3 illustrate that salts of CP-336,156 may be incorporated into a liquid reservoir patch containing a lower alkanol and skin permeation enhancers. Transdermal delivery of CP-336,156 from this formulation can be maintained for at least 7 days.

### Example 4

A transdermal liquid reservoir formulation was prepared with a solvent composition of USP alcohol (EtOH), water (H20), glycerin (Gly), glycerol monooleate (GMO), and lauryl alcohol (LA) in the proportions 30/38/30/1/1 % v/v. This mixture is a cloudy two-phase dispersion. CP-336,156 tartrate salt was added at a concentration of 6 mg/ml and the formulation was gelled with either 30 mg/g hydrophobically-modified hydroxyethylcellulose (Natrosol® Plus 330CS, Aqualon). Results of *in vitro* skin flux experiments using this liquid reservoir formulation are summarized in Table 4.

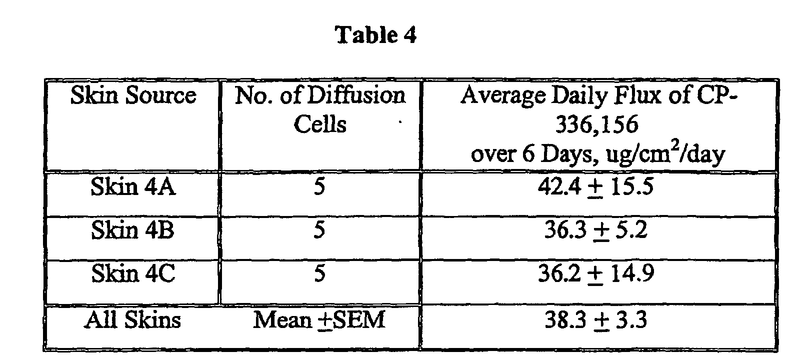

The results in Table 4 illustrate that transdermal delivery of CP-336,156 may be achieved from liquid reservoir formulations which are two-phase dispersions.

### Example 5

Transdermal liquid reservoir formulations were prepared with a solvent composition of USP alcohol (EtOH), isopropyl alcohol (IPA), water (H20), glycerin (Gly) 26.25/8.75/35/30% v/v. Permeation enhanced formulations were prepared using glycerol monooleate at concentrations of 0.03%, 0.06%, and 0.12% v/v, with the water reduced to compensate for the added enhancer. The formulations at 0%, 0.03%, and 0.06% GMO were clear solutions, while the formulation at 0.12% GMO was a cloudy dispersion. CP-336,156 tartrate salt was added at a concentration of 6 mg/ml and the formulations were gelled with 30 mg/g hydroxypropylmethylcellulose (Methocel® EIOM, Dow Chemical). Results of *in vitro* skin flux experiments using these formulations are summarized in Table 5.

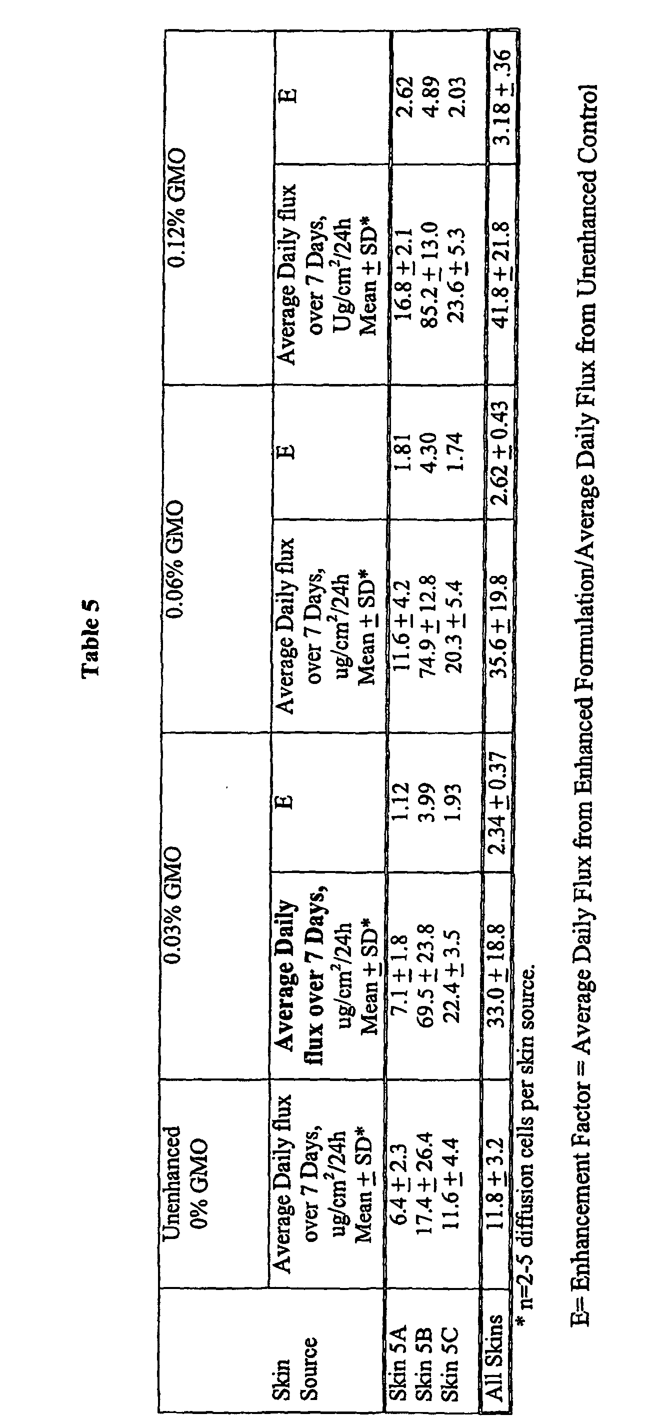

The results in Table 5 Show the addition of even very small amounts of glycerol monooleate (0.03% v/v) to a liqmd reservoir vehicle containing lower alkanols substantially increases transdermal flux of CP-336,156. These results also show that the permeation enhancement the permeation enhancement is roughly proportional to the concentration of glycerol monooleate in this range from 0.03% to 0.12%.

### Example 6

Transdermal liquid reservoir formulations were prepared with a solvent composition of EtOH/IPA/Gly/GMO 26.25/8.75/34.94%/30.00%/0.06%% v/v. CP-336,156 tartrate salt was added at 6 mg/ml and the formulation was gelled with 30 mg/g hydroxypropylmethylcellulose (Methocel® E10M, Dow Chemical). Liquid reservoir patches with 3 cm² active area were manufactured with this formulation and tested for primary dermal irritation in albino rabbits.

Each of six rabbits was exposed to an active patch (3 cm² active area). After 24 hours, the patches were removed and the sites were scored for erythema and edema at 1 and 72 hours after patch removal. The erythema and edema scores at 1 and 72 hours after removal were then averaged to give a Primary Dermal Irritation Index (PDI). PDI values were 0.3, which would classify this formulation as a barely perceptible irritant using this widelyaccepted animal model.

The preceding discussion and examples are intended merely to illustrate the art. As is apparent to one of skill in the art, various modifications can be made to the above without departing from the scope of this invention, as defined in the appended claims.

## Claims

1. A transdermal formulation comprising:
a drug reservoir;
an effective amount of lasofoxifene or a pharmaceutically acceptable salt thereof; and
an effective amount of drug permeation enhancer, wherein the drug permeation enhancer comprises an effective amount of a lower alkanol and an effective amount of glycerol monooleate.

2. A transdermal formulation according to claim 1, wherein said drug reservoir is an adhesive drug matrix reservoir.

3. A transdermal formulation according to claim 2, wherein the adhesive matrix is a solvent based pressure sensitive adhesive matrix.

4. A transdermal formulation according to claim 2, wherein the adhesive matrix is a water based pressure sensitive adhesive matrix.

5. A transdermal formulation according to claim 1, wherein said drug reservoir is a liquid reservoir drug reservoir.

6. A transdermal formulation according to any one of claims 1 to 5, wherein the effective amount of glycerol monooleate is about greater than or equal to 0.01 % w/w.

7. A transdermal formulation according to claim 1, wherein said drug reservoir is a free form hydroalcoholic gel.

8. A transdermal device comprising a transdermal formulation according to any one of claims 1 to 7 and a means for adhering the drug reservoir to an application situs.

9. A device for administering an active agent to the skin or mucosa of an individual comprising a laminated composite of:
a) a backing layer defining an upper portion of a reservoir and extending to the periphery of a peel seal disk;
b) an active agent-permeable membrane extending to the periphery of the peel seal disk and the backing layer, and underlying the backing layer, the backing layer and membrane defining;
c) the reservoir therebetween that contains the formulation according to any one of claims 1 to 7;
d) the peel seal disc underlying an active agent-permeable membrane;
e) a heat seal about the periphery of the peal seal disc, the active agent-permeable membrane and the backing layer;
f) an adhesive overlay having a central portion overlying the backing layer and a peripheral portion that extends beyond the periphery of the peel seal disc; and,
g) a removable release liner underlying the peripheral portion of the adhesive overlay and the peel seal disc.

10. A transdermal formulation according to any one of claims 1 to 7 for use in a method of treatment of the human or animal body.

11. A transdermal formulation according to any one of claims 1 to 7 for use in treatment or prevention of a disorder of the human or animal body associated with estrogen deficiency or disregulation.

12. Use of an effective amount of lasofoxifene or a pharmaceutically acceptable salt thereof and an effective amount of drug permeation enhancer, wherein the drug permeation enhancer comprises an effective amount of a lower alkanol and an effective amount of glycerol monooleate, for the preparation of a transdermal medicament for the treatment or prevention of a disorder associated with estrogen deficiency.

13. Use of an effective amount of lasofoxifene or a pharmaceutically acceptable salt thereof and an effective amount of drug permeation enhancer, wherein the drug permeation enhancer comprises an effective amount of a lower alkanol and an effective amount of glycerol monooleate, for the preparation of a transdermal medicament for use at a dermis situs for the treatment or prevention of a disorder associated with estrogen deficiency.

## Patentansprüche

1. Transdermale Formulierung, umfassend:
ein Arzneimittelreservoir;
eine wirksame Menge Lasofoxifen oder eines pharmazeutisch annehmbaren Salzes davon; und
eine wirksame Menge Arzneimittelpermeationsverstärker, wobei der Arzneimittelpermeationsverstärker eine wirksame Menge eines niederen Alkanols und eine wirksame Menge Glycerinmonooleat enthält.

2. Transdermale Formulierung nach Anspruch 1, worin das Arzneimittelreservoir ein Arzneimittel-Klebermatrixreservoir ist.

3. Transdermale Formulierung nach Anspruch 2, worin die Klebermatrix eine druckempfindliche Lösungsmittelkleber-Matrix ist.

4. Transdermale Formulierung nach Anspruch 2, worin die Klebermatrix eine druckempfindliche Klebermatrix auf wässriger Basis ist.

5. Transdermale Formulierung nach Anspruch 1, worin das Arzneimittelreservoir ein flüssiges Arzneimittelreservoir ist.

6. Transdermale Formulierung nach einem der Ansprüche 1 bis 5, worin die wirksame Menge Glycerinmonooleat 0,01 Gew.-% oder mehr beträgt.

7. Transdermale Formulierung nach Anspruch 1, worin das Arzneimittelreservoir eine freie Form eines Hydroalkoholgels ist.

8. Transdermale Vorrichtung, die eine transdermale Formulierung nach einem der Ansprüche 1 bis 7 und ein Mittel zum Aufkleben des Arzneimittelreservoirs auf eine Anwendungsstelle umfasst.

9. Vorrichtung zur Verabreichung eines Wirkstoffs an die Haut oder Mucosa einer Person, umfassend einen laminierten Verbundstoff aus:
a) einer Trägerschicht, die einen oberen Abschnitt eines Reservoirs definiert und sich bis zum Umfang einer Ablöse-Dichtungsscheibe erstreckt.
b) einer Wirkstoff-durchlässigen Membran, die sich zum Umfang der Ablöse-Dichtungsscheibe und der Trägerschicht erstreckt und unter der Trägerschicht liegt, wobei die Trägerschicht und die Membran ein Reservoir dazwischen definieren;
c) dem Reservoir dazwischen, das eine Formulierung nach einem der Ansprüche 1 bis 7 enthält;
d) der Ablöse-Dichtungsscheibe, die unter der Wirkstoff-durchlässigen Membran liegt;
e) einer Verschweißung um den Umfang der Ablöse-Dichtungsscheibe, der Wirkstoff-durchlässigen Membran und der Trägerschicht;
f) einer haftfähigen Deckschicht mit einem zentralen Abschnitt, der über der Trägerschicht liegt, und einem peripheren Abschnitt, der sich über den Umfang der Ablöse-Dichtungsscheibe hinaus erstreckt; und
g) einer abziehbaren Schutzabdeckung unter dem peripheren Abschnitt der haftfähigen Deckschicht und der Ablöse-Dichtungsscheibe.

10. Transdermale Formulierung nach einem der Ansprüche 1 bis 7 zur Verwendung in einem Verfahren zur Behandlung eines Menschen oder Tiers.

11. Transdermale Formulierung nach einem der Ansprüche 1 bis 7 zur Verwendung bei der Behandlung oder Prävention eines Leidens des menschlichen oder tierischen Körpers in Zusammenhang mit Östrogenmangel oder -dysregulation.

12. Verwendung einer wirksamen Menge Lasofoxifen oder eines pharmazeutisch annehmbaren Salzes davon und einer wirksamen Menge Arzneimittelpermeationsverstärker, wobei der Arzneimittelpermeationsverstärker eine wirksame Menge eines niederen Alkanols und einer wirksame Menge Glycerinmonooleat umfasst, zur Herstellung eines transdermalen Medikaments zur Behandlung oder Prävention eines Leidens in Zusammenhang mit Östrogenmangel.

13. Verwendung einer wirksamen Menge Lasofoxifen oder eines pharmazeutisch annehmbaren Salzes davon und einer wirksamen Menge Arzneimittelpermeationsverstärker, wobei der Arzneimittelpermeationsverstärker eine wirksame Menge eines niederen Alkanols und einer wirksame Menge Glycerinmonooleat umfasst, zur Herstellung eines transdermalen Medikaments zur Verwendung an einer Hautstelle zur Behandlung oder Prävention eines Leidens in Zusammenhang mit Östrogenmangel.

## Revendications

1. Formulation transdermique, comprenant :
un réservoir de médicament ;
une quantité efficace de lasofoxifène ou un sel pharmaceutiquement acceptable de celui-ci, et
une quantité efficace d'un agent d'augmentation de la perméation d'un médicament, où l'agent d'augmentation de la perméation d'un médicament comprend une quantité efficace d'un alcanol inférieur et une quantité efficace de monooléate de glycérol.

2. Formulation transdermique selon la revendication 1, dans laquelle ledit réservoir de médicament est un réservoir à matrice de médicament adhésive.

3. Formulation transdermique selon la revendication 2, dans laquelle la matrice adhésive est une matrice adhésive sensible à la pression, à base de solvant.

4. Formulation transdermique selon la revendication 2, dans laquelle la matrice adhésive est une matrice adhésive sensible à la pression, à base d'eau.

5. Formulation transdermique selon la revendication 1, dans laquelle ledit réservoir de médicament est un réservoir de médicament à réservoir liquide.

6. Formulation transdermique selon l'une quelconque des revendications 1 à 5, dans laquelle la quantité efficace du monooléate de glycérol est supérieure ou égale à 0,01% p/p.

7. Formulation transdermique selon la revendication 1, dans laquelle ledit réservoir de médicament est un gel hydroalcoolique sous forme libre.

8. Dispositif transdermique comprenant une formulation transdermique selon l'une quelconque des revendications 1 à 7, et un moyen pour l'adhérence du réservoir de médicament en un site d'application.

9. Dispositif pour administrer un agent actif à la peau ou les muqueuses d'un individu, comprenant un composite stratifié de :
a) une couche arrière définissant une partie supérieure d'un réservoir et se prolongeant vers la périphérie d'une disque de scellement ;
b) une membrane perméable à un agent actif, se prolongeant vers la périphérie du disque de scellement et la couche arrière, et sous-jacente à la couche arrière, la couche arrière et la membrane définissant :
c) le réservoir entre-elles qui contient la formulation selon l'une quelconque des revendications 1 à 7 ;
d) le disque de scellement, sous-jacent à une membrane perméable à l'agent actif ;
e) un scellement à chaud autour de la périphérie du disque de scellement, la membrane perméable à l'agent actif et la couche arrière ;
f) une couche adhésive ayant une partie centrale recouvrant la couche arrière et une partie périphérique qui se prolonge au-delà de la périphérie du disque de scellement, et
g) une couverture de libération éliminable, sous-jacente à la partie périphérique de la couche adhésive et au disque de scellement.

10. Formulation transdermique selon l'une quelconque des revendications 1 à 7, à utiliser dans un procédé de traitement du corps humain ou animal.

11. Formulation transdermique selon l'une quelconque des revendications 1 à 7, à utiliser dans le traitement ou la prévention d'un désordre du corps humain ou animal, associé à une déficience ou une dérégulation de l'oestrogène.

12. Utilisation d'une quantité efficace de lasofoxifène ou d'un sel pharmaceutiquement acceptable de celui-ci, et d'une quantité efficace d'un agent d'augmentation de la perméation d'un médicament, où l'agent d'augmentation de la perméation d'un médicament comprend une quantité efficace d'un alcanol inférieur et une quantité efficace de monooléate de glycérol, pour la préparation d'un médicament transdermique pour le traitement ou la prévention d'un désordre associé à une déficience en oestrogène.

13. Utilisation d'une quantité efficace de lasofoxifène ou d'un sel pharmaceutiquement acceptable de celui-ci, et d'une quantité efficace d'un agent d'augmentation de la perméation d'un médicament, où l'agent d'augmentation de la perméation d'un médicament comprend une quantité efficace d'un alcanol inférieur et une quantité efficace de monooléate de glycérol, pour la préparation d'un médicament transdermique à utiliser sur la peau pour le traitement ou la prévention d'un désordre associé à une déficience en oestrogène.
